# EUROPEAN PATENT SPECIFICATION

(11) **EP 3 752 772 B1**
(45) Date of publication and mention of the grant of the patent: **03.08.2022**
(21) Application number: 19704329.2
(22) Date of filing: 13.02.2019
(51) Int. Cl.: F24F 3/16, F24F 13/28, A61L 9/20, F24F 8/10, F24F 8/192, F24F 8/20, F24F 8/108

(54) **AIR STERILIZER UNIT**
LUFTSTERILISATIONSEINHEIT
UNITÉ DE STÉRILISATION D'AIR

(30) Priority: 14.02.2018 EP 18156783
(43) Date of publication of application: 23.12.2020
(73) Proprietor: Dolphin Care ApS, 2970 Hørsholm (DK)
(72) Inventor: TERKELSEN, Jørn, 2970 Hørsholm (DK)
(74) Representative: Kjerrumgaard, Lars Bo
(86) International application number: PCT/EP2019/053479
(87) International publication number: WO 2019/158545

(56) References cited:
- EP-A2- 1 014 006
- CN-A- 104 061 627
- JP-A- 2002 172 157
- US-A- 5 330 722
- US-A- 5 997 619
- US-A1- 2004 020 363
- US-A1- 2004 118 285

## Description

### FIELD OF THE INVENTION

The present invention relates to an air sterilizer unit for sterilizing ambient air is for instance a hospital facility. Furthermore, the present invention is concerned with a method for removing microorganisms and other polluting particles from ambient air, such as air in a hospital facility. The present invention also relates to use of such air sterilizer unit for cleaning ambient air.

### BACKGROUND OF THE INVENTION

The air in buildings and in particular rooms in buildings, such as rooms in a hospital is strongly influenced by the surroundings and the environment where polluted air is all over such buildings and in the rooms. With today's air conditioners and other air cleaning devices used indoor, the efficiency in reducing odors, and other polluting objects, including microorganisms, is poor and removing such objects and microorganisms is not possible.

US2004118285A describes an air purifier providing various functional filters within an housing, the functional filters being selected for the removal of the specific contaminants present in the environment to be purified. The functional filters comprise photocatalytic (e.g. TiO2) filters in combination with ultraviolet light sources, carbon nanotubes and HEPA filters.

CN104061627 discloses photocatalyst (TiO2) based air purifier combined with an ultraviolet light source, followed by an activated carbon and HEPA filter, all filters being disposed inside a cylinder.

JP2002172157A discloses possible sequential arrangements of functional filters, including HEPA filters, activated carbon filters and photocatalytic filters comprising titanium dioxide coatings.

### SUMMARY OF THE INVENTION

The present inventors have realized that there is a need for improving air cleaning at hospital facilities, and in particular, hospital rooms for housing sick people, and rooms for surgery. Similar needs, without limitation, are present when it comes to nurseries, kinder gardens, homes for old people, educational facilities, offices, production facilities. The ambient air at such places is polluted with microorganisms, such as bacteria, virus, fungus, and also with other particles that smells and create a poor indoor environment. The present invention is capable of reducing such odors and at the same time reduce microorganisms, and in preferred embodiments is capable of removing both orders and microorganisms from the ambient air indoor.

The invention is defined in the independent claims. The dependent claims define preferred embodiments of the invention.

The present invention concerns an air sterilizer unit having at least one inlet and at least one outlet comprising
(a) a filter housing having an interior space wherein at least one UVC light source adapted for radiating microorganisms, is located, and at least one filter with a capacity to reduce or remove air borne particles and microorganisms from ambient air, wherein the filter is UVC resistant and is located between the UVC light source and the at least one outlet,
(b) an air transportation unit, wherein the air transportation unit is adapted to move ambient air from the at least one inlet into the filter housing and being exposed to the UVC light and through the at least one filter, and through the at least one outlet.

In one preferred embodiment of the present invention the air sterilizer unit wherein a pre-filter capable of removing air borne particles having a diameter less than 200 µm is located at the inlet. Preferably, the pre-filter is capable of removing air borne particles having a diameter less than 100 µm.

In a further embodiment of the present invention the air transportation unit comprises a fan having sufficient capacity to move ambient air into the filter housing and through the at least one filter and the at least one outlet.

According to the present invention the UVC light source is adapted to provide UVC light at 250-260 nm, such as 254 nm and the at least one filter is a HEPA filter capable of removing air borne particles having a diameter less than 0.5 µm. Preferably, the HEPA filter is a HEPA14 filter capable of removing at least 99,995% of air borne particles 0.3 micrometers (µm) in diameter.

According to the present invention a second filter which is UVC resistant is located between the UVC light source and the at least one filter and comprises an active carbon filter capable of removing air borne particles having a diameter less than 100 µm.

Moreover, a third filter which is UVC resistant is located between the UVC light source and the second filter and capable of removing air borne particles having a diameter less than 100 µm, wherein the filter is coated with TiO2.

In a further preferred embodiment of the present invention the filter housing is a cylinder shaped housing. Typically, the air sterilizer unit is adapted to draw ambient air into the air transportation unit and into the filter housing to receive radiation by the UVC light and out through the at least one outlet located at the side of the cylinder housing.

In a further preferred embodiment of the present invention the UVC light source comprises between 1 and 10 UVC lamps, such as 3 or 4 UVC lamps.

In a further preferred embodiment of the present invention the HEPA filter is folded, such as pleated, to create a large surface area.

In a further preferred embodiment of the present invention the air sterilizer unit is a stand-alone device.

In a further preferred embodiment of the present invention the air sterilizer unit as a stand-alone device is suitable for functioning inside a closed room.

In another aspect the present invention relates to a method of removing microorganisms from ambient air comprising supplying current to the air sterilizer unit of the present invention and moving ambient air from the at least one inlet into the filter housing, wherein the air is being exposed to the UVC light and further moving the air exposed to UVC light through the at least one filter, and through the at least one outlet, wherein microorganisms have been removed from the air leaving the at least one outlet.

In a further aspect the present invention relates to use of an air sterilizer unit of the present invention in a room, for removing microorganisms from the room.

The present invention provides these advantages with the described solution.

Further objects and advantages of the present invention will appear from the following description, and claims.

### DESCRIPTION OF THE INVENTION

The present invention concerns an air sterilizer unit as defined in claim 1, having at least one inlet and at least one outlet comprising
(a) a filter housing having an interior space wherein at least one UVC light source adapted for radiating microorganisms, is located, and at least one filter with a capacity to reduce or remove air borne particles and microorganisms from ambient air, wherein the filter is UVC resistant and is located between the UVC light source and the at least one outlet,
(b) an air transportation unit, wherein the air transportation unit is adapted to move ambient air from the at least one inlet into the filter housing and being exposed to the UVC light and through the at least one filter, and through the at least one outlet.

The air sterilizer unit of the present invention is typically located inside a metal mesh to protect the device form injuries such as people by accident pushing or hitting the device. Also, the device is adapted to be connected to current in order to function in a house, such as a hospital, in particular a hospital room. Although the air sterilizer unit is described as two parts (a) and (b) it can come as separate parts which may then be connected at the relevant facility, or the air transportation unit may be housed at the relevant facility and the filter housing may be exchanged at certain time periods, for instance, if broken or if the capacity has gone down due to filling of the one or more filters. Also, one or more UVC lamps may be broken and needs exchange, which means separating the air sterilizer unit. In view of this the present invention also concerns a filter housing having an interior space wherein at least one UVC light source adapted for radiating microorganisms, is located, and at least one filter with a capacity to reduce or remove air borne particles and microorganisms from ambient air, wherein the filter is UVC resistant and is located between the UVC light source and the at least one outlet. It is intended that all relevant embodiments described below in relation to the filter housing of the air sterilizer unit are also embodiments of the filter housing when not connected to the air transportation unit.

Following this the present invention relates to a kit of parts comprising an air sterilizer unit having at least one inlet and at least one outlet when assembled, comprising in two parts:
(a) a filter housing having an interior space wherein at least one UVC light source adapted for radiating microorganisms, is located, and at least one filter with a capacity to reduce or remove air borne particles and microorganisms from ambient air, wherein the filter is UVC resistant and is located between the UVC light source and the at least one outlet; and
(b) an air transportation unit, wherein the air transportation unit is adapted to move ambient air from the at least one inlet into the filter housing and being exposed to the UVC light and through the at least one filter, and through the at least one outlet.

In one embodiment of the present invention the air sterilizer unit wherein a pre-filter capable of removing air borne particles having a diameter less than 200 µm is located at the inlet. Preferably, the pre-filter is capable of removing air borne particles having a diameter less than 100 µm, such as between 10 and 100 µm. Typically, the pre-filter is a class G3 filter with Arrestance A (5) 80 < A < 90. Examples of particles to be removed are drops, dirt, visible dust.

In a further embodiment of the present invention the air transportation unit comprises a fan having sufficient capacity to move ambient air into the filter housing and through the at least one filter and the at least one outlet.

The UVC light source is adapted to provide UVC light at 100-280 nm, such as from 250-260 nm, such as 254 nm. The UVC light at 250-260 nm as claimed in present invention, provides a much more effective killing of bacteria and virus while at the same time the UCV light does not destroy the material of the air sterilizer unit, in particular the various filters as defined.

The at least one filter is a HEPA filter capable of removing air borne particles having a diameter less than 0.5 µm. Preferably, the HEPA filter is a HEPA14 filter capable of removing 99,995% of air borne particles 0.3 micrometers (µm) in diameter. HEPA stands for high efficiency particulate air and is sometimes also referred to as high-efficiency particulate arresting or high-efficiency particulate arrestance.

Moreover, according to the present invention a second filter which is UVC resistant is located between the UVC light source and the at least one filter and comprises an active carbon filter capable of removing air borne particles having a diameter less than 100 µm, such as between 10 and 100 µm. Typically, the carbon filter is a class G4 filter with Arrestance A (5) 90 < A. Examples of particles are visible dust. Typically, the carbon filter has a surface area of between 0.5 to 2 m², such as from 0.2 to 1.5 m². When the second filter is present the ambient air to be cleaned first travels into the filter housing where it is radiated and the through the second filter and then through the at least first filter and out via the at least one outlet.

In the present invention a third filter which is UVC resistant is located between the UVC light source the second filter and capable of removing air borne particles having a diameter less than 100 µm, such as between 10 and 100 µm, wherein the filter is coated with TiO2. Typically, the third filter is a glass filter which is coated with TiO2, such as a class M5 filter with efficiency of E (%) 40 < E < 60. Examples of particles to be removed are visible dust and pollen. Typically, the TiO₂ coated filter has a surface area of between 0.1 to 1.5 m², such as from 0.2 to 0.5 m². When the third filter is present the ambient air to be cleaned first travels into the filter housing where it is radiated and then through the third filter, and hereafter travels through the second filter and then through the at least first filter and out via the at least one outlet.

The term "arrestance" as used herein is a measure for how efficient the filter removes larger particles.

In a further embodiment of the present invention the filter housing is a cylinder shaped housing. Typically, the air sterilizer unit is adapted to draw ambient air into the air transportation unit and into the filter housing to receive radiation by the UVC light and out through the at least one outlet located at the side of the cylinder housing. Other constructions of such a filter house is possible such as an ellipse shaped construction. In a typical embodiment the cylinder shaped housing is a cartridge filter housing.

In a further embodiment of the present invention the UVC light source comprises between 1 and 10 UVC lamps. In reality only, the size of the available space inside the filter housing sets the limits for UVC lamps, but for a stand-alone unit, it is preferable to have from 1-10 lamps. In one embodiment the UVC light source is 3 UVC lamps. In another embodiment the UVC light source is 4 UVC lamps. Preferably, the UVC lamps are located so that the distance between each light source is the same.

In a further embodiment of the present invention the HEPA filter is folded, such as pleated, to create a large surface area. Typically, the HEPA filter is folded to create a surface area of between 1 to 20 m², such as from 5 to 15 m².

In a further embodiment of the present invention the air sterilizer unit is a stand-alone device.

In a further embodiment of the present invention the air sterilizer unit as a stand-alone device is suitable for functioning inside a closed room.

A further aspect the present invention as defined in claim 11 relates to a method of removing microorganisms from ambient air comprising supplying current to the air sterilizer unit of the present invention and any one of the defined embodiments and moving ambient air from the at least one inlet into the filter housing, wherein the air is being exposed to the UVC light and further moving the air exposed to UVC light through the at least one filter, and through the at least one outlet, wherein microorganisms and other particulate material have been removed from the air leaving the at least one outlet.

A further aspect the present invention as defined in claim 12 relates to use of an air sterilizer unit of the present invention and any one of the defined embodiments in a room, for removing microorganisms and other particulate material from ambient air from the room.

The term "stand-alone" as used herein means a device which can be transported by the user, such as people working at a hospital, and plugged-in (for current supply) at any desired place, such as in a hospital room for sick people, a room for surgery, an office in a building.

The invention will now be described more fully with reference to the appended drawings illustrating typical embodiments of the air sterilizer unit of the present invention.

These drawings are by no means limiting the scope of the present invention and are only intended to guide the skilled person for better understanding of the present invention.

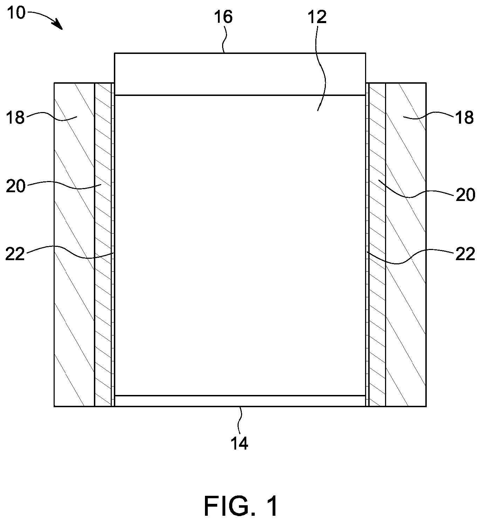

Figure 1 illustrates a cross/side section side-view of a cartridge filter device (10) having an interior space or chamber (12) wherein at least one UVC is located (not shown). The filter device has a cylindric housing with a top (14) closed and non-permeable to air, and a bottom flange (16) which is adapted to receive an air transportation unit in accordance with the present invention. The outer circumference of the filter device (10) is a HEPA filter (18) which can remove microorganisms. In close proximity and surrounded by the first filter (18) is a second carbon filter (20) which can remove odors and other particulate material, when the air is pushed through. The second filter (20) is in close proximity to a third filter (22) typically a glass filter coated with TiO₂, and surrounds such filter (22). The third filter (22) surrounds a space (12) and is the filter that is directly exposed to UVC light when the device (10) is in operation.

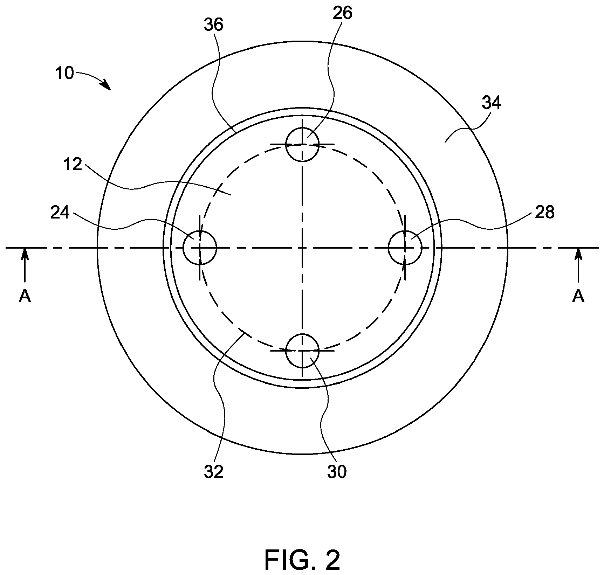

Figure 2 illustrates a top view cross section of the cartridge filter device (10), and shows its cylindrical shape. Inside the cartridge filter device (10) is a space or chamber (12), wherein 4 UVC lamps (24, 26, 28, 30) are located with the same distance from each other and placed on a circumference (32). As explained in figure 1 the first filter, typically a HEPA14 filter, and optionally a second carbon filter (34) defines an outer circumference and a third filter (36) defines the inner circumference and surrounds the space (12).

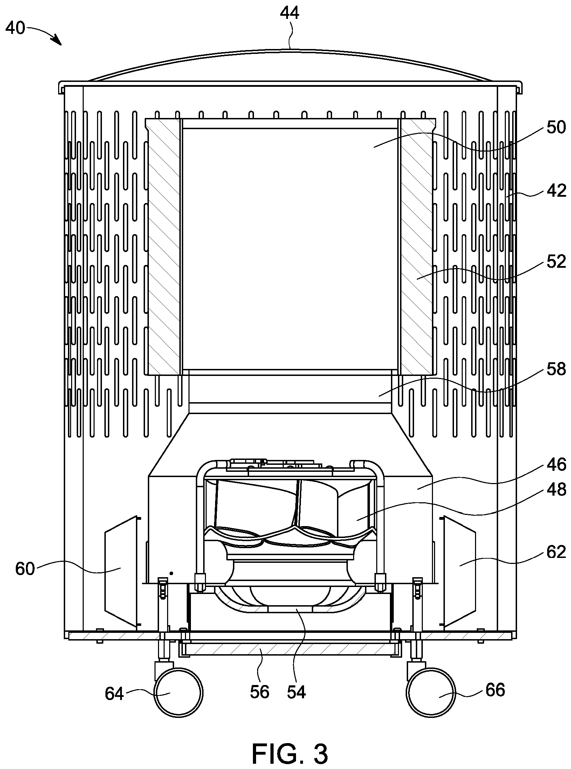

Figure 3 illustrates an embodiment of the cartridge filter (40) of the air sterilizer device of the present invention , , protected by a cover panel (42) having a top cover (44) and wherein the cover panel (42) extends down to protect an air transportation unit (46) such as a fan house having a fan (48) adapted to draw air from the surroundings into the device (40). Inside the cover panel (42) is the cartridge filter device and the fan house (46) with the fan (48) wherein the fan when in operation draws ambient air into the chamber (50) and out through the one or more filters (52) and further out a metal mesh (not shown) at the sides of the cartridge filter (40). UVC lamps (not shown) are located inside the filter chamber (50). The bottom flange (58) of the cartridge filter house fits into a corresponding flange with the fan chamber (46) holding the two pieces together in air tight mode. Ballasts (60, 62) are placed on each side of the fan house (46) and may be facing the fan house and/or may be facing the cover panel (42). At the bottom of the fan house (46) is a flow grid (54) and ambient air is being drawn into the fan house (46) through a pre-filter (56). The cartridge filter device (40) is equipped with wheels (64, 66) to make it easy to move for a person working at for instance a hospital.

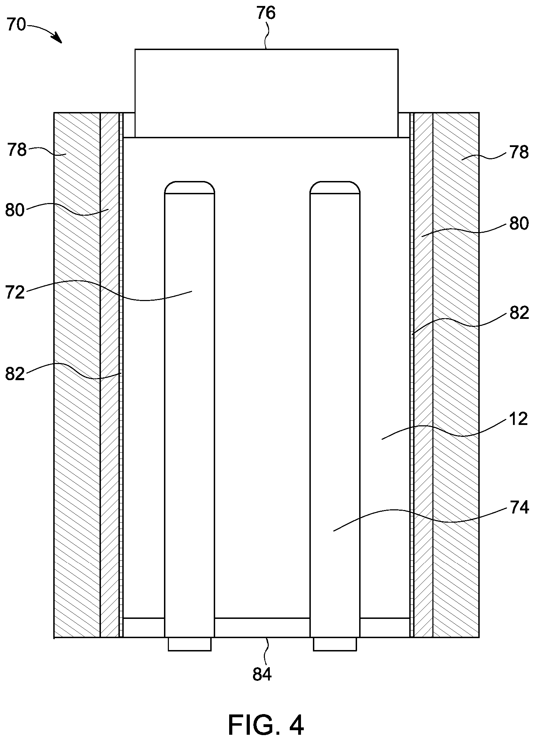

Figure 4 illustrates a side-view cross section of the cartridge filter (70) of the air sterilizer device of the present invention, wherein the UVC lamps (72, 74) are shown. The filter (70) has an interior chamber (12) wherein at least one UVC is located (72, 74). The filter device has a cylindric house with a top (84) closed and non-permeable to air, and a bottom flange (76) which is adapted to receive an air transportation unit in accordance with the present invention. The outer circumference of the filter device (70) is typically a HEPA filter (78) which can remove microorganisms. In close proximity and surrounded by the first filter (78) is a second carbon filter (80) which can remove odors and other particulate material, when the air is pushed through. The second filter (80) is in close proximity to a third filter (82) typically a glass filter coated with TiO₂ and surrounds such filter (82). The third filter (82) surrounds the space (12) and is the filter that is directly exposed to UVC light (72, 74) when the device (70) is in operation.

All headings and sub-headings are used herein for convenience only and should not be construed as limiting the invention in any way.

Recitation of ranges of values herein are merely intended to serve as a short method of referring individually to each separate value falling within the range, unless other-wise indicated herein, and each separate value is incorporated into the specification as if it were individually recited herein. Unless otherwise stated, all exact values provided herein are representative of 5 corresponding approximate values (e.g., all exact exemplary values provided with respect to a particular factor or measurement can be considered to also provide a corresponding approximate measurement, modified by "about", where appropriate).

The terms "a" and "an" and "the" and similar referents as used in the context of describing the invention are to be construed to insert both the singular and the plural, unless otherwise indicated herein or clearly contradicted by context. Thus, "a" and "an" and "the" may mean at least one, or one or more.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

Throughout the description when "selected from" or "selected from the group consisting of' is used it also means all possible combinations of the stated terms, as well as each individual term.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context (*e.g*., a composition described herein as comprising a particular element should be understood as also describing a composition consisting of that element, unless otherwise stated or clearly contradicted by context).

The features disclosed in the foregoing description provide support to the invention as defined in the appended claims.

### EXPERIMENTALS

The air sterilizer unit of the present invention as a cartridge filter device (see figures 1-3) have been made in two versions, a smaller version with an air intake of 500 m3/hour during the day and 250 m3/hour during the night, and a larger version with an air intake of 1000 m3/hour during the day and 500 m3/hour during the night.

The smaller version has a 6.7 m² HEPA 14 filter, a 1.0 m² carbon filter and a 0.28 m² TiO₂ coated filter. Also, there are three UVC lamps.

The larger version has a 14.2 m² HEPA 14 filter, a 1.554 m² carbon filter and a 0.42 m² TiO₂ coated filter. Also, there are four UVC lamps.

Both versions when current is applied operates by taking a large amount of ambient air (see details above) into the cartridge filter device, and by means of a rotating fan (backward curved centrifugal fan; B-wheel) the ambient air is further pushed through the different filters and out through the sides of the cartridge filter device.

The large cartridge filter device has a diameter of 470 mm and a height of 400 mm. In one version this filter is composed of three filter materials, wherein one is a HEPA14 filter with a surface area of 14.2 m², the second is a 1.554 m² carbon filter and the third is a glass based filter coated with 0.28 m² TiO₂. There are 4 UVC lamps each with a radiation wavelength of 254 nm.

It has been observed that the use of a glass based filter coated with TiO₂ leads to higher efficiency in killing microorganisms, and without being bound by theory it is contemplated that when titanium dioxide (TiO₂) is irradiated with UVC light a photocatalytic reaction takes place. Strong oxidizing hydroxyls radicals is formed. The radicals destroy the cell wall of the bacteria. Titanium dioxide (TiO₂) function as a catalyst in the process and is therefore not consumed over time.

In the present experiment such cartridge filter is placed in a hospital room wherein ambient air is drawn into the cartridge filter from the bottom through a pre-filter (class G3) comprising polyester fibers. Hereafter, the air is lead through the ventilator house with the centrifugal fan (air transportation unit) and further up and entering the space of the cartridge filter wherein the UVC lamps radiates the air and kills microorganisms. The air wherein microorganisms and other pollutant particles have been removed then returns to the room as clean and sterilized air.

In another experiment a cartridge filter having three filters is tested. As explained above the air is drawn into the space of the cartridge filter wherein the UVC lamps radiates the air and kills microorganisms. There are 4 UVC lamps producing a radiation wavelength of 254 nm, and each lamp has an intensity of 98 µW/cm² and the total intensity is 392 µW/cm². After being radiated the air is first lead through a glass based filter coated with TiO₂, and then the air is pushed through a second filter based on active carbon which absorbs odors and other particulate pollutants. Finally, the air is pushed through the HEPA14 filter which is folded/pleated to remove microorganisms and has a filtration efficiency of 99.995% of airborne particles (bacteria and virus) of 0.3 µm in diameter. The cleaned air is then recirculated into to surrounding space.

Experiments with this cartridge filter device to test the active carbon filter gave the following results, wherein efficiency is indicated by numbers 1 to 4.

| Table showing efficiency of activated carbon filter for various agents. | | | | | |
|---|---|---|---|---|---|
| 4 = very good adsorption, 3 = good adsorption, 2 = acceptable adsorption, 1 = reduced adsorption | | | | | |
| acetone | 3 | Fatty acids | 4 | carbon dioxide CO2 | - |
| Acetaldehyde | 4 | chlorin | 1 | chemical solvent | 4 |
| acrolein | 1 | chloroform | 4 | menthol | 4 |
| alcohol | 4 | diesel oil mist | 3 | methane | 1 |
| anaesthetics | 3 | acetic acid | 4 | methanol | 3 |
| ether | 3 | disinfectant | 4 | merkaptane | 2 |
| essential oil | 4 | formaldehyd | 2 | phenol | 4 |
| ethane | 1 | Fruit odor | 4 | phosgene | 3 |
| ethylen | 1 | Household odor | 4 | propane | 2 |
| ethyl acetate | 4 | iodine | 4 | Candy smell | 4 |
| amines | 2 | cerosine | 4 | tetrachlorcarbons | 4 |
| ammonia | 1 | sweat | 4 | turpentine | 4 |
| gasoline | 4 | cosmetic | 4 | Tobacco odor | 4 |
| benzene | 4 | hospital odor | 4 | toluol | 4 |
| buthane | 2 | cresol | 4 | | |

The ability to handle large amounts of air (1.000 m³/hour) demands a large filter area and by folding as a pleated filter a very large surface area of 14.2 m² have been created in this large cartridge filter device having a diameter of 450 mm and a height of 400 mm. Experiments with an air sterilizer unit (cartridge filter) having a diameter of 560 mm and a height of 560 mm of the present invention with 4 UVC lambs producing a radiation wavelength of 254 nm and wherein the filter for reducing microorganisms had a surface area of 20.16 m2 and is a M6 with efficiency E (%) 60 < E < 80 (removing particles with diameter of 10-25 µm) showed 30-40 % reduction in microbial counts compared to the situation without filter but regular cleaning..

## Claims

1. An air sterilizer unit (40) having at least one inlet and at least one outlet comprising
(a) a filter housing having an interior space (12,50) wherein at least one UVC light (24,74) source adapted for radiating microorganisms, is located, and at least one filter with a capacity to reduce or remove microorganisms from ambient air, wherein the filter is UVC resistant and is located between the UVC light source and the at least one outlet,
(b) an air transportation unit (46),
wherein the air transportation unit is adapted to move ambient air from the at least one inlet into the filter housing and being exposed to the UVC light and through the at least one filter, and through the at least one outlet,
wherein the UVC light source is adapted to provide UVC light at 250-260 nm,
wherein the at least one filter (52) is a HEPA filter (18,78) capable of removing air borne particles having a diameter less than 0.5 µm,
wherein a second filter (20,80) which is UVC resistant is located between the UVC light source and the at least one filter and comprises an active carbon filter capable of removing air borne particles having a diameter less than 100 µm,
wherein a third filter (22,82) which is UVC resistant is located between the UVC light source and the second filter and is capable of removing air borne particles having a diameter less than 100 µm, wherein the filter is coated with TiO₂.

2. The air sterilizer unit of claim 1 wherein a pre-filter (56) capable of removing air borne particles having a diameter less than 200 µm, is located at the inlet.

3. The air sterilizer unit of claim 1 or 2 wherein the air transportation unit comprises a fan (48) having sufficient capacity to move ambient air into the filter housing and through the at least one filter and the at least one outlet.

4. The air sterilizer unit of any one of claims 1-3 wherein the at least one filter is a HEPA 14 filter.

5. The air sterilizer unit of any one of claims 1-4 wherein the filter housing is a cylinder shaped housing.

6. The air sterilizer unit of any one of claims 1-5 wherein the UVC light source is between 1 and 10 UVC lamps, such as 3-4 UVC lamps.

7. The air sterilizer unit of any one of claims 5-6 adapted to draw ambient air into the air transportation unit and into the filter housing to receive UVC light and out through the at least one outlet located at the side of the cylinder housing.

8. The air sterilizer unit of any one of claims 1-7 wherein the HEPA filter is folded, to create a large surface area.

9. The air sterilizer unit of any one of claims 1-8 which is a stand-alone device.

10. The air sterilizer unit of any one of claims 1-9 which is suitable for functioning inside a closed room.

11. A method of removing microorganisms from ambient air comprising supplying current to the air sterilizer unit of any one of claims 1-10 and moving ambient air from the at least one inlet into the filter housing, wherein the air is being exposed to the UVC light and further moving the air exposed to UVC light through the at least one filter, and through the at least one outlet, wherein microorganisms have been removed from the air leaving the at least one outlet.

12. Use of an air sterilizer unit of any one of claims 1-10 in a room, for removing microorganisms from the room.

## Patentansprüche

1. Eine Luftsterilisatoreinheit (40), die mindestens einen Einlass und mindestens einen Auslass hat, umfassend
(a) ein Filtergehäuse mit einem Innenraum (12, 50), in dem sich mindestens eine UVC-Lichtquelle (24, 74) befindet, die zum Bestrahlen von Mikroorganismen angepasst ist, und mindestens einen Filter mit einer Fähigkeit, Mikroorganismen aus der Umgebungsluft zu reduzieren oder zu entfernen, wobei der Filter UVC-beständig ist und sich zwischen der UVC-Lichtquelle und dem mindestens einen Auslass befindet,
(b) eine Lufttransporteinheit (46), wobei die Lufttransporteinheit dafür ausgelegt ist, Umgebungsluft von dem mindestens einen Einlass in das Filtergehäuse, wo diese dem UVC-Licht ausgesetzt wird, und durch den mindestens einen Filter und durch den mindestens einen Auslass zu bewegen,
wobei die UVC-Lichtquelle dafür ausgelegt ist, UVC-Licht bei 250 bis 260 nm bereitzustellen,
wobei der mindestens eine Filter (52) ein HEPA-Filter (18, 78) ist, der in der Lage ist, luftgetragene Partikel mit einem Durchmesser von weniger als 0,5 µm zu entfernen,
wobei ein zweiter Filter (20, 80), welcher UVC-beständig ist, zwischen der UVC-Lichtquelle und dem mindestens einen Filter angeordnet ist und einen Aktivkohlefilter umfasst, der in der Lage ist, luftgetragene Partikel mit einem Durchmesser von weniger als 100 µm zu entfernen,
wobei ein dritter Filter (22, 82), welcher UVC-beständig ist, zwischen der UVC-Lichtquelle und dem zweiten Filter angeordnet ist und in der Lage ist, luftgetragene Partikel mit einem Durchmesser von weniger als 100 µm zu entfernen, wobei der Filter mit TiO₂ beschichtet ist.

2. Die Luftsterilisatoreinheit des Anspruchs 1, wobei sich am Einlass ein Vorfilter (56) befindet, der in der Lage ist, luftgetragene Partikel mit einem Durchmesser von weniger als 200 µm zu entfernen.

3. Die Luftsterilisatoreinheit des Anspruchs 1 oder 2, wobei die Lufttransporteinheit einen Lüfter (48) mit ausreichender Kapazität umfasst, um Umgebungsluft in das Filtergehäuse und durch den mindestens einen Filter und den mindestens einen Auslass zu bewegen.

4. Die Luftsterilisatoreinheit von einem der Ansprüche 1 bis 3, wobei der mindestens eine Filter ein HEPA-14-Filter ist.

5. Die Luftsterilisatoreinheit von einem der Ansprüche 1 bis 4, wobei das Filtergehäuse ein zylinderförmiges Gehäuse ist.

6. Die Luftsterilisatoreinheit von einem der Ansprüche 1 bis 5, wobei die UVC-Lichtquelle aus zwischen 1 und 10 UVC-Lampen, wie etwa 3 bis 4 UVC-Lampen, besteht.

7. Die Luftsterilisatoreinheit von einem der Ansprüche 5 bis 6, die geeignet ist, Umgebungsluft in die Lufttransporteinheit und in das Filtergehäuse einzusaugen, um UVC-Licht zu empfangen, und sie durch den mindestens einen Auslass, der sich an der Seite des Zylindergehäuses befindet, herauszusaugen.

8. Die Luftsterilisatoreinheit von einem der Ansprüche 1 bis 7, wobei der HEPA-Filter gefaltet ist, um einen großen Oberflächeninhalt zu erzeugen.

9. Die Luftsterilisatoreinheit von einem der Ansprüche 1 bis 8, welche eine eigenständige Vorrichtung ist.

10. Die Luftsterilisatoreinheit von einem der Ansprüche 1 bis 9, welche zum Betrieb innerhalb eines geschlossenen Raums geeignet ist.

11. Ein Verfahren zum Entfernen von Mikroorganismen aus Umgebungsluft, umfassend das Zuführen von Strom zu der Luftsterilisatoreinheit von einem der Ansprüche 1 bis 10 und das Bewegen von Umgebungsluft von dem mindestens einen Einlass in das Filtergehäuse, worin die Luft dem UVC-Licht ausgesetzt wird und weiteres Bewegen der dem UVC-Licht ausgesetzten Luft durch den mindestens einen Filter und durch den mindestens einen Auslass, wobei Mikroorganismen aus der den mindestens einen Auslass verlassenden Luft entfernt worden sind.

12. Verwendung von einer Luftsterilisatoreinheit von einem der Ansprüche 1 bis 10 in einem Raum, um Mikroorganismen aus dem Raum zu entfernen.

## Revendications

1. Une unité de stérilisation d'air (40) ayant au moins une entrée et au moins une sortie comprenant
(a) un logement de filtre ayant un espace intérieur (12, 50) dans lequel au moins une source de lumière UVC (24, 74) adaptée pour rayonner des micro-organismes est située, et au moins un filtre avec une capacité à réduire ou éliminer les micro-organismes de l'air ambiant, dans lequel le filtre est résistant aux UVC et est située entre la source de lumière UVC et la au moins une sortie,
(b) une unité de transport d'air (46), dans laquelle l'unité de transport d'air est adaptée pour déplacer l'air ambiant de la au moins une entrée dans le boîtier de filtre, y étant exposée à la lumière UVC, et à travers le au moins un filtre, et à travers la au moins une sortie,
dans laquelle la source de lumière UVC est adaptée pour fournir de la lumière UVC à 250-260 nm,
dans laquelle le au moins un filtre (52) est un filtre HEPA (18, 78) capable d'éliminer les particules en suspension dans l'air ayant un diamètre inférieur à 0,5 µm,
dans laquelle un deuxième filtre (20, 80) qui est résistant aux UVC est situé entre la source de lumière UVC et le au moins un filtre et comprend un filtre à charbon actif capable d'éliminer les particules en suspension dans l'air ayant un diamètre inférieur à 100 µm,
dans laquelle un troisième filtre (22, 82) qui est résistant aux UVC est situé entre la source de lumière UVC et le deuxième filtre et est capable d'éliminer les particules en suspension dans l'air ayant un diamètre inférieur à 100 µm, dans laquelle le filtre est recouvert de TiO₂.

2. L'unité de stérilisation d'air de la revendication 1, dans laquelle un préfiltre (56) capable d'éliminer les particules en suspension dans l'air ayant un diamètre inférieur à 200 µm, est situé à l'entrée.

3. L'unité de stérilisation d'air selon la revendication 1 ou 2, dans laquelle l'unité de transport d'air comprend un ventilateur (48) ayant une capacité suffisante pour déplacer l'air ambiant dans le boîtier de filtre et à travers le au moins un filtre et la au moins une sortie.

4. L'unité de stérilisation d'air de l'une quelconque des revendications 1 à 3, dans laquelle le au moins un filtre est un filtre HEPA 14.

5. L'unité de stérilisation d'air de l'une quelconque des revendications 1 à 4, dans laquelle le boîtier de filtre est un boîtier en forme de cylindre.

6. L'unité de stérilisation d'air de l'une quelconque des revendications 1 à 5, dans laquelle la source de lumière UVC est comprise d'entre 1 et 10 lampes UVC, par exemple 3-4 lampes UVC.

7. L'unité de stérilisation d'air de l'une quelconque des revendications 5 à 6 adaptée pour aspirer de l'air ambiant dans l'unité de transport d'air et dans le boîtier de filtre pour recevoir de la lumière UVC et le sortir à travers la au moins une sortie située dans le côté du boîtier cylindrique.

8. L'unité de stérilisation d'air de l'une quelconque des revendications 1 à 7, dans laquelle le filtre HEPA est plié, afin de créer une grande aire de surface.

9. L'unité de stérilisation d'air de l'une quelconque des revendications 1 à 8, qui est un dispositif autonome.

10. L'unité de stérilisation d'air de l'une quelconque des revendications 1 à 9, qui est apte à fonctionner à l'intérieur d'une pièce fermée.

11. Un procédé d'élimination de micro-organismes de l'air ambiant comprenant l'alimentation en courant de l'unité de stérilisation d'air de l'une quelconque des revendications 1 à 10 et le déplacement de l'air ambiant de la au moins une entrée dans le boîtier de filtre, dans lequel l'air est exposé à la lumière UVC, et déplacer en outre l'air exposé à la lumière UVC à travers le au moins un filtre, et à travers la au moins une sortie, dans lequel les micro-organismes ont été éliminés de l'air quittant l'au moins une sortie.

12. Utilisation d'une unité de stérilisation d'air de l'une quelconque des revendications 1 à 10 dans une pièce, pour éliminer des micro-organismes de la pièce.
